# EUROPÄISCHE PATENTSCHRIFT

(11) **EP 3 405 225 B1**
(45) Veröffentlichungstag und Bekanntmachung des Hinweises auf die Patenterteilung: **30.03.2022**
(21) Anmeldenummer: 17700816.6
(22) Anmeldetag: 17.01.2017
(51) Int. Cl.: A61M 1/36

(54) **BLUTBEHANDLUNGSVORRICHTUNG UND ORGANIZER**
BLOOD TREATMENT DEVICE AND ORGANIZER
DISPOSITIF DE TRAITEMENT DU SANG ET ORGANISEUR

(30) Priorität: 20.01.2016 DE 102016100934
(43) Veröffentlichungstag der Anmeldung: 28.11.2018
(73) Patentinhaber: Fresenius Medical Care Deutschland GmbH, 61352 Bad Homburg (DE)
(72) Erfinder: SCHRÖRS, Alexander, 60389 Frankfurt am Main (DE); KLEWINGHAUS, Jürgen, 61440 Oberursel (DE)
(74) Vertreter: Bobbert & Partner Patentanwälte PartmbB
(86) Internationale Anmeldenummer: PCT/EP2017/050875
(87) Internationale Veröffentlichungsnummer: WO 2017/125382

(56) Entgegenhaltungen:
- WO-A2-2009/146913
- US-A1- 2005 070 837
- US-A1- 2010 121 246
- US-A1- 2012 075 266
- US-A1- 2013 165 847

## Beschreibung

Die Erfindung betrifft eine Blutbehandlungsvorrichtung gemäß Anspruch 1 und einen Organizer gemäß Anspruch 11.

Aus der Praxis sind Vorrichtungen zur extrakorporalen Blutbehandlung, insbesondere zur Dialysebehandlung, bekannt, mittels welcher wahlweise unterschiedliche Behandlungsverfahren bzw. Behandlungsoptionen ausgeführt werden können. Dabei ist für unterschiedliche Behandlungsoptionen ein jeweils unterschiedlicher Umfang an Blutschlauchsätzen (Disposables) und zugehörigen Blutschlauchsatz-Komponenten, wie z. B. Schlauchverbindungen und dergleichen, erforderlich.

Aus dem Stand der Technik sind Zusammenstellungen von BlutschlauchsatzKomponenten zum Bilden von Blutschläuchen bekannt, aus welchen sich Blutschläuche bzw. Behandlungssets für unterschiedliche Behandlungsoptionen zusammenstellen lassen. Solche Zusammenstellungen, wie sie bspw. aus der WO 2009/146913 A2 oder der US 2010/121246 bekannt sind, können auf sogenannten "Organizern" oder "Trays" übersichtlich und für die Ankoppel-Gegebenheiten der verwendeten Blutbehandlungsvorrichtung passend vorgehalten werden. Je nach durchzuführender Behandlungsoption können die hierzu benötigten Blutschlauchsatz-Komponenten entsprechend konnektiert werden. Die für die ausgewählte Behandlungsoption nicht benötigten Blutschlauchsatz-Komponenten verbleiben ungenutzt am Organizer.

Ein hiermit verbundener Nachteil besteht darin, dass die Vielzahl der am Organizer vorliegenden Blutschlauchsatz-Komponenten sowie deren vielfältige Verbindungsmöglichkeiten miteinander verwirrend auf das Klinikpersonal wirkt, weshalb es regelmäßig und insbesondere unter Zeitdruck zu fehlerhafter Auswahl und Konnektierung von Blutschlauchsatz-Komponenten kommt. Zudem erfordert das Vorbereiten eines einsatzfähigen Blutschlauchsatzes aus den am Organizer vorhandenen Blutschlauchsatz-Komponenten für die ausgewählte Behandlungsoption Erfahrung und ist überdies zeitaufwändig, da oft mehrere Schlauchsätze oder Schlauchleitungen gewissenhaft verfolgt werden müssen, um eine fehlerhafte Verbindung und Verwechslungen zu vermeiden und dabei eine große Anzahl ähnlich aussehender Blutschlauchsatz-Komponenten im Blick behalten werden muss.

Eine erfindungsgemäße Aufgabe kann darin bestehen, einen weiteren Organizer, welcher Komponenten, insbesondere Blutschlauchsatz-Komponenten, für die Blutbehandlung trägt, vorzuschlagen. Zudem soll eine Blutbehandlungsvorrichtung angegeben werden, welche den Organizer trägt oder hierzu vorbereitet ist.

Die Aufgabe kann gelöst werden durch eine Blutbehandlungsvorrichtung mit den Merkmalen des Anspruchs 1 und durch einen Organizer mit den Merkmalen des Anspruchs 11.

Erfindungsgemäß wird somit eine Blutbehandlungsvorrichtung vorgeschlagen, welche wenigstens je eine Steuervorrichtung, ein User Inferface und einen Aufnahmeabschnitt aufweist.

Der Aufnahmeabschnitt dient dem lösbaren Aufnehmen eines Organizers, welcher lösbar mit ihm verbundene Komponenten, insbesondere Blutschlauchsatz-Komponenten, trägt. Einige oder alle dieser Komponenten sind für ein extrakorporales Blutbehandlungsverfahren bestimmt, welches mittels der Blutbehandlungsvorrichtung durchführbar ist.

Die Blutbehandlungsvorrichtung weist wenigstens eine Vorrichtung zum Einwirken auf Komponenten eines im Aufnahmeabschnitt lösbar aufgenommenen Organizers auf. Diese Vorrichtung ist vorzugsweise im Bereich des Aufnahmeabschnitts angeordnet oder diesem zugeordnet.

Die Blutbehandlungsvorrichtung weist optional ferner wenigstens eine Vorrichtung zum lösbaren Befestigen des Organizers an ihr, insbesondere am Aufnahmeabschnitt, auf. Erfindungsgemäß wird ferner ein Organizer vorgeschlagen mit wenigstens einer hieran angeordneten Befestigungseinrichtung, mittels welcher wenigstens eine Komponente, insbesondere eine Blutschlauchsatz-Komponente, lösbar an einem weiteren Abschnitt des Organizers, etwa an einem Rücken hiervon, gehalten wird.

Der weitere Abschnitt und/oder die Befestigungseinrichtung können optional eine Öffnung aufweisen, durch welche eine Vorrichtung zum Einwirken auf Komponenten mit der Komponente und/oder der Befestigungseinrichtung in Kontakt gebracht werden kann, beispielsweise durch Abschnitte der Befestigungseinrichtung hindurch.

Die Befestigungseinrichtung kann optional mittels lösbaren Klebstoffs mit einem weiteren Abschnitt des Organizers, etwa dessen Rücken, verbunden sein.

Die Befestigungseinrichtung kann optional einen Verbinder aufweisen, welcher mit einer Vorrichtung zum Einwirken auf Komponenten der Blutbehandlungsvorrichtung verbindbar und hierfür ausgestaltet ist.

Die Befestigungseinrichtung und/oder eine lösbar im Organizer aufgenommene Komponente können optional ein magnetisches Element aufweisen.

Erfindungsgemäße Ausführungsformen können eines oder mehrere der vorstehend oder im Folgenden genannten Merkmale in beliebiger Kombination aufweisen, sofern ihre Kombination für den Fachmann nicht erkennbar technisch unmöglich ist. Erfindungsgemäße Ausführungsformen sind ferner Gegenstand der Unteransprüche.

Bei allen hierin gemachten Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

In bestimmten, beispielhaften erfindungsgemäßen Ausführungsformen kann, wenn hierin von einer Komponente die Rede ist auch eine Kombination von, insbesondere miteinander verbundenen, Komponenten verstanden werden.

In bestimmten, beispielhaften erfindungsgemäßen Ausführungsformen dient der Organizer zum lösbaren Aufnehmen und/oder lösbaren Fixieren von Komponenten von Blutschläuchen für unterschiedliche extrakorporale Blutbehandlungsoptionen, insbesondere bei Dialyseverfahren, mit einer Blutbehandlungsvorrichtung, wie bspw. einer Dialysevorrichtung. Geeignete Einrichtungen zum lösbaren Aufnehmen und/oder Fixieren solcher Komponenten am Organizer sind am Organizer vorgesehen.

In bestimmten, beispielhaften erfindungsgemäßen Ausführungsformen weist der Organizer einen einstückigen Hauptkörper auf. Dieser kann die Einrichtungen zum lösbaren Aufnehmen und/oder lösbaren Fixieren tragen oder aufweisen.

In bestimmten, beispielhaften erfindungsgemäßen Ausführungsformen ist der Organizer aus Polystyrol gefertigt oder weist solches auf.

In bestimmten, beispielhaften erfindungsgemäßen Ausführungsformen ist der Organizer u. a. mittels Thermoformen hergestellt.

In bestimmten, beispielhaften erfindungsgemäßen Ausführungsformen kann das User Inferface eine Eingabevorrichtung und/oder eine Ausgabevorrichtung sein oder aufweisen. Eine Ausgabevorrichtung kann ein Display, ein Monitor oder ein Bildschirm sein. Eine Eingabevorrichtung kann eine Tastatur, ein Touch-Screen oder dergleichen sein.

Die Steuervorrichtung kann konfiguriert oder programmiert sein, um eine oder mehrere Komponenten des Organizers und/oder ein Aufrüsthandbuch oder eine Anleitung zum Aufrüsten der Komponenten an der Blutbehandlungsvorrichtung anzuzeigen.

In bestimmten, beispielhaften erfindungsgemäßen Ausführungsformen ist die Vorrichtung zum Einwirken auf Komponenten ausgestaltet oder angeordnet, um nicht direkt auf eine Komponente einzuwirken sondern auf eine Befestigungseinrichtung des Organizers, mittels welcher die betreffende Komponente lösbar am Organizer, z. B. an einem Rücken hiervon, gehalten wird. Die "Vorrichtung zum Einwirken auf Komponenten" ist in bestimmten, beispielhaften erfindungsgemäßen Ausführungsformen daher auch als Vorrichtung zu verstehen, welche auf die Befestigungseinrichtung, welche die Komponente lösbar am Organizer hält, einwirkt.

In bestimmten, beispielhaften erfindungsgemäßen Ausführungsformen ist eine Komponente eine Blutschlauchsatz-Komponente. Die vorliegende Erfindung ist hierauf jedoch nicht beschränkt. Ist hierin von einer Komponente die Rede, so kann dies unvermindert auch auf eine Blutschlauchsatz-Komponente übertragen werden, und umgekehrt.

Erfindungsgemäß ist die Vorrichtung zum Einwirken auf Komponenten des Organizers eine Vorrichtung zum Freigeben von Komponenten des Organizers, eine Vorrichtung zum aktiven Trennen von Komponenten vom Organizer und/oder eine Vorrichtung zum Halten von Komponenten am Organizer oder weist eine solche auf.

Unter "Freigeben" wird in bestimmten, beispielhaften erfindungsgemäßen Ausführungsformen verstanden, dass die (freigegebene) Komponente ab dem Moment der Freigabe (nicht aber zuvor) auf übliche Weise manuell durch den Benutzer vom Organizer entnommen werden kann (also beispielsweise ohne zerstörerisch tätig sein zu müssen oder ohne Einsatz von Werkzeug). Ein "Freigeben" kann ein passives Lösen erlauben.

Unter "Trennen" wird in bestimmten, beispielhaften erfindungsgemäßen Ausführungsformen verstanden, dass die (getrennte) Komponente ab dem Moment der Trennung (nicht aber zuvor) nicht mehr manuell vom Benutzer vom Organizer gelöst werden kann, da sie mit diesem bereits nicht mehr verbunden ist. Ein "Trennen" kann wie ein "Auswerfen" ein aktives Lösen sein.

Unter "Halten" wird in bestimmten, beispielhaften erfindungsgemäßen Ausführungsformen verstanden, dass die (gehaltene) Komponente bis zu dem Moment, in welchem das Halten beendet wird, nicht auf übliche Weise manuell vom Benutzer vom Organizer entnommen werden kann (also beispielsweise nicht ohne zerstörerisch tätig sein zu müssen oder nicht ohne Einsatz von Werkzeug). Erst nach Beendigung des Haltezustands kann die Komponente manuell entnommen werden.

In bestimmten, beispielhaften erfindungsgemäßen Ausführungsformen ist die Vorrichtung zum Einwirken auf Komponenten des Organizers als wenigstens ein mechanisch wirkendes Bauteil ausgestaltet oder weist ein solches auf.

Erfindungsgemäße Beispiele für ein mechanisch wirkendes Bauteil umfassen insbesondere Vorrichtungen zum Auswerfen der Komponente, Vorrichtungen zum Öffnen eines Riegels des Organizers, Drehvorrichtungen zum Entriegeln eines Bajonettverschlusses oder einer Lasche des Organizers, Vorrichtungen zum Eröffnen eines Hinterschnitts des Organizers und/oder Vorrichtungen zum Spreizen oder Verklemmen zweier Abschnitte des Organizers, wobei letztere angeordnet sind, um insbesondere auf die dem Aufnahmeabschnitt oder der Blutbehandlungsvorrichtung zugewandte Seite des im Aufnahmeabschnitt aufgenommenen Organizers einzuwirken.

In bestimmten, beispielhaften erfindungsgemäßen Ausführungsformen ist die Vorrichtung zum Einwirken auf Komponenten des Organizers als eine Energiequelle oder als Emitter zum Emittieren von Wellen, Licht und/oder Wärme, und/oder als eine Magnetfeldquelle ausgestaltet oder weist eine solche auf.

In bestimmten, beispielhaften erfindungsgemäßen Ausführungsformen ist die Steuervorrichtung konfiguriert und/oder programmiert, um die Vorrichtung zum Einwirken auf Komponenten derart zu aktivieren oder anzusteuern, dass diese auf die wenigstens eine Komponente einwirkt, welche vom Organizer getragen wird, welcher im Aufnahmeabschnitt lösbar aufgenommen ist.

In bestimmten, beispielhaften erfindungsgemäßen Ausführungsformen, ist die Steuervorrichtung konfiguriert und/oder programmiert, um auf Komponenten des Organizers in Abhängigkeit einer mittels des User Interface dargestellten oder bezeichneten Komponente einzuwirken. Das Einwirken erfolgt mittels der Vorrichtung zum Einwirken auf Komponenten. "ln Abhängigkeit" kann sich in bestimmten, beispielhaften erfindungsgemäßen Ausführungsformen auf die Reihenfolge, mit welcher die Komponenten mittels User Interface dargestellt oder bezeichnet werden, beziehen.

In solchen Ausführungsformen wird also "in derselben Reihenfolge" auf die Komponenten eingewirkt, in welcher sie auch mittels User Inferface, z. B. am Bildschirm, gezeigt oder bezeichnet werden. Zeigt das User Interface etwa zunächst eine erste, dann eine zweite und schließlich eine dritte Komponente auf einem Bildschirm (etwa beim Lesen einer Anleitung zum Aufrüsten der Komponenten an der Blutbehandlungsvorrichtung), so wird auch mittels der Vorrichtung zum Einwirken auf Komponenten zunächst auf die erste Komponente, dann auf die zweite und schließlich auf die dritte Komponente eingewirkt.

So können beispielsweise Komponenten des Organizers in einer festgelegten Reihenfolge freigegeben oder ausgeworfen werden, welche jener Reihenfolge entspricht, in welcher die freigegebenen oder ausgeworfenen Komponenten dem Benutzer am User Interface, etwa beim Studieren der Anleitung zum Aufrüsten der Blutbehandlungsvorrichtung angezeigt werden. Exemplarisch sei hier eine Programmierung genannt, bei welcher der Benutzer auf einer bestimmten Seite der Anleitung zum Aufrüsten angewiesen wird, den Alphachip der Blutpumpe an der Blutbehandlungsvorrichtung einzulegen und ihm mittels der Steuervorrichtung der Alphachip freigegeben oder ausgeworfen wird. Blättert der Benutzer am User Interface virtuell in der Anleitung zum Aufrüsten weiter und wird er auf einer späteren Seite aufgefordert, eine nächste Komponente aufzurüsten, so wird ihm - z. B. zeitgleich zum Anzeigen der nächste Komponente am Bildschirm - mittels der Steuervorrichtung diese nächste Komponente freigegeben oder ausgeworfen.

In manchen erfindungsgemäßen Ausführungsformen ist die Steuervorrichtung programmiert, um bei Anzeigen einer bestimmten Komponente des Organizers auf dem User Interface auf genau diese Komponente mittels der Vorrichtung zum Einwirken einzuwirken, etwa durch deren Freigabe oder Auswurf.

Eine solche Ausgestaltung kann als reine User Interface-Steuerung bezeichnet werden.

Eine solche Ausgestaltung muss sich selbstredend nicht auf eine erste und eine zweite Komponente beschränken sondern kann sich auf jede auf dem Organizer vorhandene Komponente oder eine Reihenfolge von Komponenten erstrecken. Dies gilt optional allgemein stets dann, wenn hierin von "erster" und "zweiter" Komponente die Rede ist.

In bestimmten, beispielhaften erfindungsgemäßen Ausführungsformen weist die Blutbehandlungsvorrichtung wenigstens eine Detektionsvorrichtung auf oder ist hiermit in Signalverbindung verbunden, welche ihrerseits mit der Steuervorrichtung in Signalverbindung steht. Die Detektionsvorrichtung ist konfiguriert, um eine vorliegende Verbindung einer vorbestimmten ersten Komponente mit der Blutbehandlungsvorrichtung zu detektieren, also um festzustellen, dass oder ob die erste Komponente mit der Blutbehandlungsvorrichtung verbunden wurde.

In bestimmten, beispielhaften erfindungsgemäßen Ausführungsformen ist die Steuervorrichtung konfiguriert und/oder programmiert, bei Detektion der erfolgten Verbindung der ersten vorbestimmten Komponente (also wenn sie erkennt, dass eine Verbindung zwischen der ersten Komponente und der Blutbehandlungsvorrichtung hergestellt wurde) mittels der Vorrichtung zum Einwirken auf Komponenten auf eine vorbestimmte zweite Komponente einzuwirken. Das Einwirken kann ein Freigeben sein. Alternativ ist die Steuervorrichtung in manchen, beispielhaften erfindungsgemäßen Ausführungsformen konfiguriert und/oder programmiert, solange auf eine vorbestimmte zweite Komponente einzuwirken, und zwar wiederum mittels der Vorrichtung zum Einwirken auf Komponenten, bis mittels der Detektionsvorrichtung eine erfolgte Verbindung der ersten vorbestimmten Komponente erkannt wurde ("bis zur", also bis sie erkennt, dass eine Verbindung zwischen der ersten Komponente und der Blutbehandlungsvorrichtung hergestellt wurde). Das Einwirken kann dann ein Halten sein.

So können beispielsweise Komponenten stets dann freigegeben oder ausgeworfen werden, wenn erkannt wurde, dass eine vorangegangene Komponente (die Reihenfolge, in welcher die Komponenten zu verbinden sind, kann der Steuervorrichtung zugänglich hinterlegt oder gespeichert sein) mit der Blutbehandlungsvorrichtung verbunden wurde. Wird beispielsweise der Citratpumpenclip in die Citrattropfkammer eingelegt, so löst dies ein Auswerfen der Citratleitung aus dem Organizer aus.

Eine solche Ausgestaltung kann als reine Ereignis-Steuerung bezeichnet werden.

In bestimmten, beispielhaften erfindungsgemäßen Ausführungsformen ist die Steuervorrichtung konfiguriert und/oder programmiert, um den Benutzer mittels des User Interface zum Verbinden der Blutbehandlungsvorrichtung mit einer vorbestimmten Komponente des Organizers aufzufordern. Zur Überprüfung, dass der Benutzer die konkrete Komponente mit der Blutbehandlungsvorrichtung verbunden hat, werden entsprechende Signale einer Detektionsvorrichtung abgewartet oder ausgewertet.

In diesen Ausführungsformen wird der Benutzer mittels User Interface zum Verbinden der Blutbehandlungsvorrichtung mit einer vorbestimmten weiteren Komponente des Organizers aufgefordert, wenn mittels der Detektionsvorrichtung eine erfolgte Verbindung der ersten vorbestimmten Komponente oder ein erfolgtes Entnehmen der ersten vorbestimmten Komponente aus dem Organizer erkannt wurde. Auf diese vorbestimmte weitere Komponente des Organizers wird mittels Vorrichtung zum Einwirken auf Komponenten eingewirkt. Das Einwirken kann ein Freigeben sein. Alternativ ist die Steuervorrichtung in manchen, beispielhaften erfindungsgemäßen Ausführungsformen konfiguriert und/oder programmiert, solange auf eine vorbestimmte zweite Komponente einzuwirken, und zwar mittels der Vorrichtung zum Einwirken auf Komponenten, bis mittels der Detektionsvorrichtung eine erfolgte Verbindung oder ein erfolgtes Entnehmen der ersten vorbestimmten Komponente erkannt wurde ("bis zur", also bis sie erkennt, dass eine Verbindung zwischen der ersten Komponente und der Blutbehandlungsvorrichtung hergestellt wurde). Das Einwirken kann dann ein Halten sein.

So können beispielsweise Komponenten stets dann freigegeben oder ausgeworfen werden, wenn erkannt wurde, dass eine vorangegangene Komponente (die Reihenfolge, in welcher die Komponenten zu verbinden sind, kann der Steuervorrichtung zugänglich hinterlegt oder gespeichert sein), zu deren Verbindung der Benutzer in einem vorangegangenen Schritt aufgefordert wurde, tatsächlich mit der Blutbehandlungsvorrichtung verbunden wurde. Hiermit verbunden kann ein automatisches, virtuelles Weiterblättern in der Anleitung zum Aufrüsten mit entsprechender Darstellung am User Interface einhergehen.

Eine solche Ausgestaltung kann als Kombination aus User-Interface-Steuerung und Ereignis-Steuerung bezeichnet werden.

In bestimmten, beispielhaften erfindungsgemäßen Ausführungsformen weist die Blutbehandlungsvorrichtung wenigstens eine Detektionsvorrichtung auf, welche mit der Steuervorrichtung in Signalverbindung steht. Die Detektionsvorrichtung ist konfiguriert, eine vorliegende Verbindung einer zur Blutbehandlung erforderlichen Einrichtung, bei welcher es sich nicht um eine der Komponenten des Organizers handeln muss, mit der Blutbehandlungsvorrichtung zu detektieren. Alternativ oder ergänzend ist die Detektionsvorrichtung konfiguriert, zu detektieren, dass eine vorbestimmte, zum Ausführen einer zur Blutbehandlung erforderliche Handlung oder ein Vorgang, etwa ein Test wie der T1-Test, ein Druckhaltetest oder dergleichen erfolgt ist.

Die Steuervorrichtung ist in diesen Ausführungsformen konfiguriert und/oder programmiert, um Signale der Detektionsvorrichtung hinsichtlich der erfolgten Verbindung der Einrichtung oder der erfolgten Handlung/des erfolgten Vorgangs auszuwerten. Die Steuervorrichtung ist in diesen Ausführungsformen ferner konfiguriert und/oder programmiert, bei Detektion der erfolgten Verbindung der Einrichtung oder der erfolgten Handlung oder des erfolgten Vorgangs mittels der Vorrichtung zum Einwirken auf Komponenten des Organizers auf eine vorbestimmte Komponente des Organizers einzuwirken. Das Einwirken kann dann ein Freigeben sein. Alternativ ist die Steuervorrichtung in manchen, beispielhaften erfindungsgemäßen Ausführungsformen konfiguriert und/oder programmiert, solange auf eine vorbestimmte Komponente einzuwirken, und zwar mittels der Vorrichtung zum Einwirken auf Komponenten, bis mittels der Detektionsvorrichtung die erfolgte Verbindung oder die erfolgte Handlung/der erfolgte Vorgang detektiert wurde. Das Einwirken kann dann ein Halten sein.

In bestimmten, beispielhaften erfindungsgemäßen Ausführungsformen ist die Blutbehandlungsvorrichtung lösbar mit einem Organizer verbunden, welcher seinerseits lösbar mit ihm verbundene Komponenten trägt.

In bestimmten, beispielhaften erfindungsgemäßen Ausführungsformen ist die Blutbehandlungsvorrichtung eine Hämodialysevorrichtung, eine Hämodiafiltrationsvorrichtung, eine Hämofiltrationsvorrichtung und/oder eine Apharesevorrichtung.

Einige oder alle erfindungsgemäßen Ausführungsformen können einen oder mehrere der oben oder im Folgenden genannten Vorteile aufweisen.

Ein Vorteil besteht darin, dass, wenn das Einwirken ein Halten ist, verhindert werden kann, dass der Benutzer bestimmte Komponenten zu früh mit der Blutbehandlungsvorrichtung verbindet. So kann verhindert werden, dass Komponenten, die nicht Gegenstand der Überprüfung der Blutbehandlungsvorrichtung, etwa im Rahmen des sog. T1-Tests, welche diesen Test jedoch stören könnten, nicht vor Abschluss des T1-Tests verbunden werden. Dies kann durch Halten der Komponente, was deren Entnahme vom Organizer und ihr anschließendes Verbinden verhindert, erzielt werden.

Ein Halten kann ferner vorteilhaft sicherstellen, dass für eine beabsichtigte Blutbehandlungsoption überzählige, also nicht erforderliche, Komponenten des Organizers bis zum Abschluss des Aufrüstens der Blutbehandlungsvorrichtung oder darüber hinaus nicht vom Organizer entnommen werden können. Auf diese Weise kann sichergestellt werden, dass überzählige Komponenten nicht versehentlich mit aufgerüstet werden.

Ferner kann dem Benutzer vorteilhaft eine Unterstützung hinsichtlich der Reihenfolge, in welcher die auf dem Organizer vorliegenden Komponenten mit der Blutbehandlungsvorrichtung verbunden werden müssen, gegeben werden; die Steuervorrichtung gibt die Komponenten in der korrekten Reihenfolge frei, falls gewünscht. Dass sich die einzelnen Komponenten hierbei verheddern, überschneiden oder kreuzen, kann durch ihre geschickte Anordnung auf dem Organizer vorab, aber auch durch Halten und/oder ihre Freigabe/Auswerfen in der vorgegebenen, optimierten Reihenfolge zusätzlich ausgeschlossen werden.

Das gezielte Freigeben/Auswerfen oder Halten kann ferner einen Vorteil bieten, da der Benutzer immer nur einzelne Komponenten zu beachten und zu verbinden hat; die Steuervorrichtung gibt ihm vor, welche Komponente dies zum jeweiligen Zeitpunkt ist. Dies dient der Übersichtlichkeit, wodurch die hohe Anzahl an Komponenten für den Benutzer beherrschbar gemacht wird.

Insbesondere durch das aktive Auswerfen von Komponenten kann die vom Benutzer für ein Lösen der Komponenten vom Organizer aufzubringende Kraft gering gehalten werden.

Die vorliegende Erfindung wird im Folgenden anhand der beigefügten Zeichnungen, in welcher identische Bezugszeichen gleiche oder ähnliche Bauteile bezeichnen, exemplarisch erläutert. In den zum Teil stark vereinfachten Figuren gilt:
- Fig. 1: zeigt einen exemplarischen erfindungsgemäßen Organizer in einer Draufsicht;
- Fig. 2: zeigt den Organizer der Fig. 1, wobei er in Fig. 2 anders als in Fig. 1 nicht mit Blutschlauchsatz-Komponenten bestückt ist;
- Fig. 3: zeigt eine Befestigungseinrichtung des erfindungsgemäßen Organizers gemäß einer ersten Ausführungsform in Ansicht von vorne, ohne Blutschlauchsatz-Komponenten;
- Fig. 4: zeigt eine Befestigungseinrichtung des erfindungsgemäßen Organizers gemäß einer zweiten Ausführungsform in Ansicht von vorne, ohne Blutschlauchsatz-Komponenten;
- Fig. 5: zeigt eine Befestigungseinrichtung des erfindungsgemäßen Organizers gemäß einer dritten Ausführungsform in perspektivischer Ansicht von vorne, mit Blutschlauchsatz-Komponenten, in einem Haltezustand;
- Fig. 6: zeigt die Befestigungseinrichtung der Fig. 5 in einem Freigabezustand; und
- Fig. 7: zeigt schematisch vereinfacht eine erfindungsgemäße Blutbehandlungsvorrichtung in einer exemplarischen Ausführungsform.

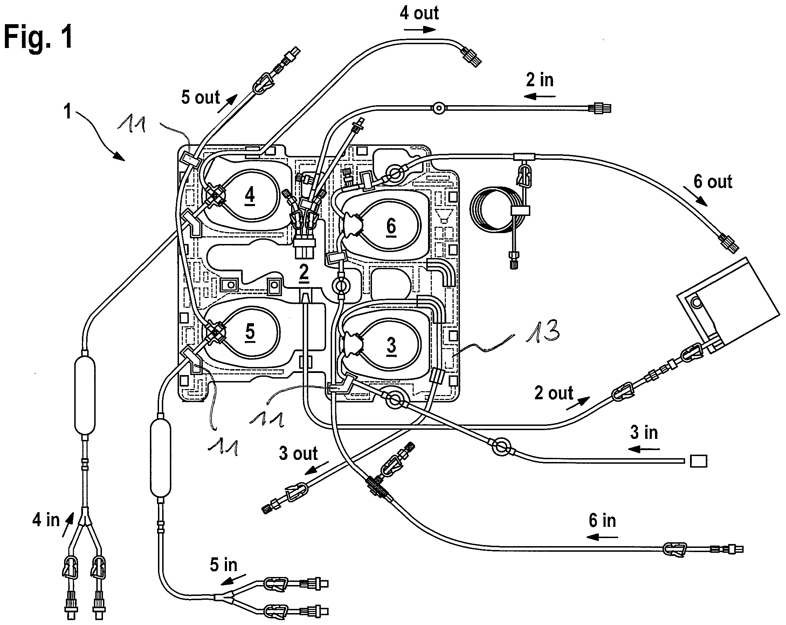

**Fig. 1** zeigt schematisch vereinfacht einen exemplarisch bestückten erfindungsgemäßen Organizer 1 (auch als Tray bezeichnet), welcher bestückt ist mit einer Rückgabeleitung 2 zur Rückgabe von Blut von einem nicht dargestellten Filter zum Patienten, einer Filtratleitung 3 vom Filter zu einem nicht dargestellten Filtratbeutel, einer Dialysatleitung 4 für Dialysierflüssigkeit oder Austauschflüssigkeit von einem nicht dargestellten Lösungsbeutel zum Filter oder einem Anschlussstück, einer Substituatleitung 5 für Austauschflüssigkeit vom Lösungsbeutel zum Anschlussstück und einer Zugangsleitung 6 vom Patienten zum Filter oder der Rückgabeleitung 2.

Dabei umfasst die Rückgabeleitung 2 einen Filterkonnektor 2in sowie einen Patientenkonnektor 2out mit Spülbeutel. Die Filtratleitung 3 umfasst einen Filterkonnektor 3in sowie einen Konnektor 3out für den Filtratbeutel. Die Dialysatleitung 4 umfasst einen Konnektor 4in für den Lösungsbeutel und einen Filterkonnektor 4out für ein Anschlussstück in der Zugangs- oder Rückgabeleitung. Die Substituatleitung 5 umfasst einen Konnektor 5in für den Lösungsbeutel sowie einen Konnektor 5out für das Anschlussstück in der Zugangs- oder Rückgabeleitung. Die Zugangsleitung 6 vom Patienten zum Filter umfasst einen Patientenkonnektor 6in sowie einen Filterkonnektor 6out.

Die vorgenannten Elemente mit Bezugszeichen 2 bis 6 stellen Blutschlauch- oder Blutschlauchsatzkomponenten dar und sind Beispiele für Komponenten, welche lösbar an einem erfindungsgemäßen Organizer 1 angebracht sein können.

Der Organizer 1 weist Befestigungseinrichtungen 11 auf, welche dem lösbaren Verbinden der Blutschlauchsatz-Komponenten mit einem Rücken 13 (meist aus einem mehr oder weniger steifen Material) oder einem anderen Abschnitt des Organizers 1 dienen.

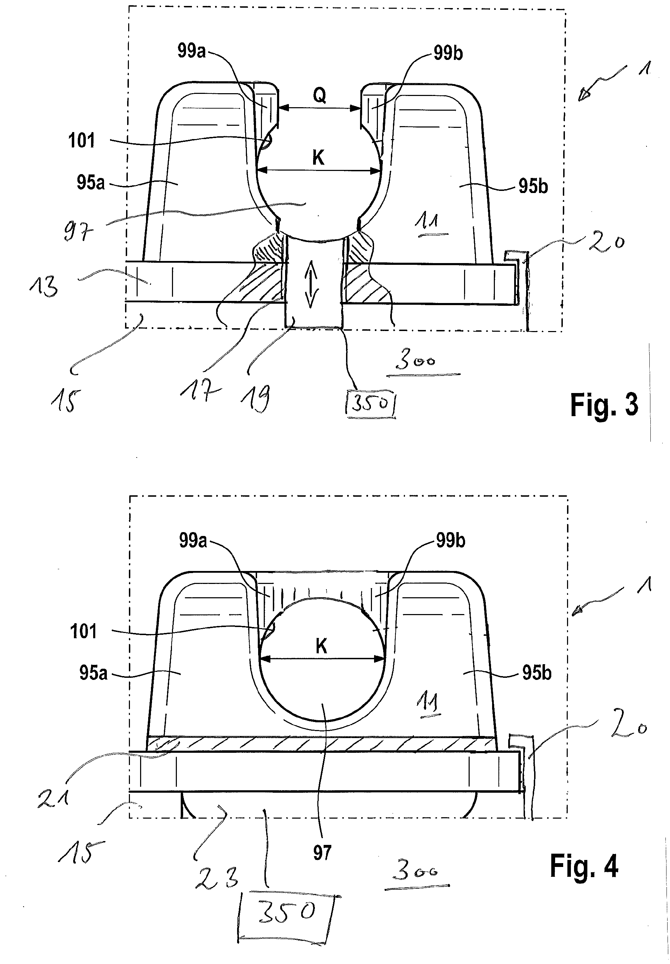

Fig. 1 ist nicht zu entnehmen, dass mehrere der Befestigungsvorrichtungen 11 Öffnungen wie jene aufweisen, welche in Fig. 3 das Bezugszeichen 17 tragen und optional durch sowohl durch den Rücken 13 als auch durch Abschnitte der Befestigungsvorrichtung 11 hindurch ragen.

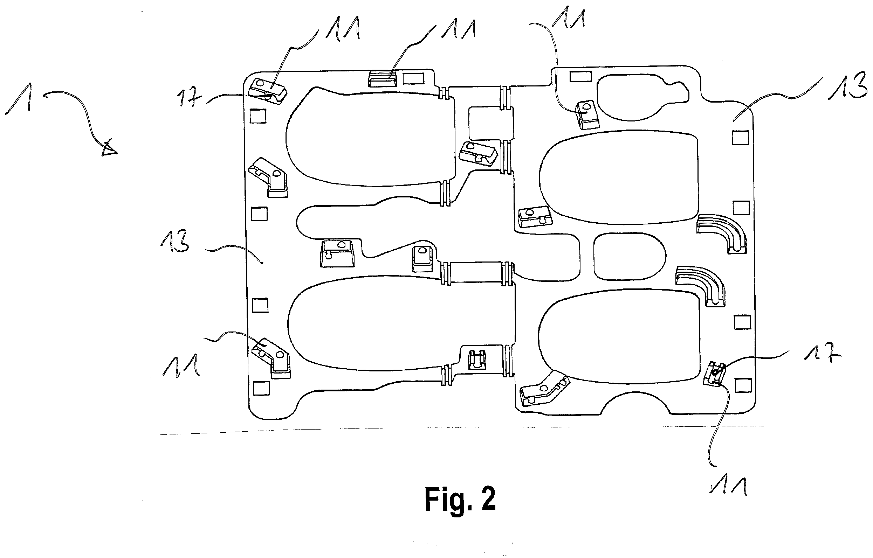

**Fig. 2** zeigt den Organizer 1 der Fig. 1, wobei er in Fig. 2 nicht mit BlutschlauchsatzKomponenten bestückt ist, in einer Draufsicht.

In Fig. 2 angedeutete Öffnungen 17 sind mit Bezug auf Fig. 3 erläutert.

**Fig. 3** zeigt eine Befestigungseinrichtung 11 des erfindungsgemäßen Organizers 1 gemäß einer ersten Ausführungsform in Ansicht von vorne. Die Befestigungseinrichtung 11 trägt keine Blutschlauchsatz-Komponente.

Die Befestigungseinrichtung 11 ist als Clip ausgestaltet. Sie weist zwei Schenkel 95a und 95b auf, welche jeweils einen (in Fig. 3) nach oben sowie an seinen Stirnseiten offenen Kanal 97 seitlich begrenzen. Der somit teil- oder halboffen ausgestaltete Kanal 97 dient der Aufnahme einer in Fig. 3 nicht dargestellten Blutschlauchsatz-Komponente.

Die Schenkel 95a und 95b haben jeweils einen in Richtung der oberen Öffnung mit der Weite Q des Kanals 97 weisenden Vorsprung bzw. eine Verstärkung 99a bzw. 99b. Mittels der Verstärkungen 99a und 99b kann eine Klemmwirkung auf eine in die Befestigungseinrichtung 11 eingelegte Blutschlauchsatz-Komponente (in Fig. 3 nicht gezeigt) erzielt werden.

Die Weite Q der Öffnung, welche geringer als eine Weite K des Kanals 97 ist, in welchem die Blutschlauchsatz-Komponente innerhalb der Befestigungseinrichtung 11 zum Liegen kommt, bietet eine erhöhte Sicherheit dafür, dass sich die Blutschlauchsatz-Komponente nicht unbeabsichtigt aus der Befestigungseinrichtung 11 löst.

Unterhalb des Kanals 97 ist der steife Rücken 13 des Organizers 1 teilweise geschnitten dargestellt. Man erkennt, dass der steife Rücken 13 unterhalb des Kanals 97 eine Öffnung 17 aufweist, welche sich auch durch Abschnitte der Befestigungsvorrichtung 11 erstreckt, welche dem Rücken 13 anliegen.

Zu erkennen ist ferner, dass der steife Rücken 13 des Organizers 1 einem Aufnahmeabschnitt 15 einer Blutbehandlungsvorrichtung 300 anliegt. Der Aufnahmeabschnitt 15 dient der Aufnahme des Organizers 1 an der Blutbehandlungsvorrichtung 300 und weist in der exemplarischen Ausführungsform der Fig. 3 einen Auswerfer 19 auf. Der Auswerfer 19 ist eine exemplarische Ausgestaltung der Vorrichtung zum Einwirken auf Komponenten eines im Aufnahmeabschnitt lösbar aufgenommenen Organizers 1.

Der Auswerfer 19 ist angeordnet, um entlang der Richtungen des Doppelpfeils der Fig. 3 verschoben werden zu können. Wird er, bezogen auf Fig. 3, nach oben verschoben, so drückt er auf oder gegen die durch den Kanal 97 verlaufende, in Fig. 3 nicht gezeigte Blutschlauchsatz-Komponente und zwängt diese auf diese Weise durch die Öffnung mit der Weite Q hindurch nach oben. Auf diese Weise trennt der Auswerfer 19 die nicht gezeigte Blutschlauchsatz-Komponente aktiv vom Organizer 1. Er wirft sie aus.

Der Auswerfer 19 steht in Signalverbindung mit einer Steuervorrichtung 350 der Blutbehandlungsvorrichtung 300 und kann von dieser zum Auswerfen aktiviert werden.

Für den Fachmann ist erkennbar, dass es keiner Öffnung 17 im Sinne einer Durchgangsöffnung bedarf, durch welche der Auswerfer 19 (oder die Vorrichtung zum Einwirken allgemein) hindurch geführt wird. Der Auswerfer 19 kann vielmehr auch durch den geschlossenen Rücken 13 hindurch drücken und auf diese Weise auswerfen. Der Rücken 13 kann hierzu einen gegenüber benachbarten Abschnitten des Rückens 13 dünneren Abschnitt aufweisen, über welchen unter Aufbringen einer vergleichsweise nur geringen Kraft mittels Auswerfer 19 auf die Komponente eingewirkt wird.

Das Bezugszeichen 20 bezeichnet eine Vorrichtung zum lösbaren Fixieren des Organizers 1, z. B. mittels dessen Rückens 13, an der Blutbehandlungsvorrichtung 300, hier exemplarisch am Aufnahmeabschnitt 15. Die Vorrichtung 20 kann als Widerlager verstanden werden, mittels welchem die Befestigungseinrichtungen 11 relativ zur Vorrichtung zum Einwirken gehalten wird. Sie kann ferner dazu dienen, eine Ausweichbewegung des Organizers 1 beim Einwirken auf die Befestigungseinrichtung, etwa mittels Auswerfer 19, zu verhindern.

Mit dem Bezugszeichen 101 ist eine optionale Detektionsvorrichtung bezeichnet, welche z. B. als Drucksensor, optischer Sensor oder dergleichen ausgestaltet ist. Die Detektionsvorrichtung 101 kann vorgesehen und mit der Steuervorrichtung 350 verbunden sein. Ihre Rückmeldung kann eine Aussage darüber erlauben, ob die betreffende Komponente bereits aus der Befestigungseinrichtung 11 des Organizers 1 entnommen wurde. Diese Aussage kann Voraussetzung dafür sein, dass weitere Komponenten freigesetzt oder ausgeworfen werden. Die Steuervorrichtung 350 kann entsprechend programmiert sein.

**Fig. 4** zeigt eine Befestigungseinrichtung 11 des erfindungsgemäßen Organizers 1 gemäß einer zweiten Ausführungsform in Ansicht von vorne und wiederum ohne Blutschlauchsatz-Komponente.

Anders als in der Ausführungsform der Fig. 3 ist der Kanal 97 optional nicht nach oben durch eine Öffnung mit der Weite Q (vergleiche Fig. 3) geöffnet (gleichwohl kann auch die Ausgestaltung der Fig. 4 eine solche Öffnung aufweisen). Der Kanal 97 weist anders als in Fig. 3 optional auch keine Öffnung zum Einführen eines Auswerfers 19 auf (vergleiche Fig. 3). Der Kanal 97 kann eine oder beide dieser Öffnungen haben; er kann alternativ aber auch wie in Fig. 4 gezeigt, entlang seines Umfangs geschlossen sein.

Anders als im Ausführungsbeispiel der Fig. 3 weist die Blutbehandlungsvorrichtung 300 optional auch keinen Auswerfer 19 auf.

Die Befestigungseinrichtung 11 der Fig. 4 ist mittels einer im Schnitt gezeigten Klebeschicht 21 am steifen Rücken 13 oder anderer Stelle des Organizers 1 verbunden. Die Klebeschicht 21 kann einen lösbaren, z. B. mittels Wärme oder mittels UV-Licht lösbaren Klebstoff enthalten oder hieraus bestehen.

Die Blutbehandlungsvorrichtung 300 weist einen Licht- oder Wärmestrahler 23 auf, welcher Teil des Aufnahmeabschnitts 15 sein kann. Der Licht- oder Wärmestrahler 23 kann ein UV-Strahler sein. Die von ihm abgegebene Strahlung dient dazu, die Klebeschicht 21 zu lösen, so dass sich die Befestigungseinrichtung 11 vom Rücken 13 löst.

Im Beispiel der Fig. 4 verbleibt die vom Rücken 13 gelöste Befestigungseinrichtung 11 als Ganzes an der Blutschlauchsatz-Komponente. Die in der Befestigungseinrichtung 11 gehaltene Blutschlauchsatz-Komponente bleibt somit mit der Befestigungseinrichtung 11 verbunden, nicht aber mit dem Rücken 13.

Es ist offensichtlich, dass die Erfindung auch solche Ausführungsformen umfassen soll, bei welchen nur ein Teil der Befestigungseinrichtung 11 mittels Klebeschicht 21 mit dem Rücken 13 oder anderen Teilen des Organizers 1 verbunden ist. Wird die Klebeschicht 21 mittels des Licht- oder Wärmestrahler 23 gelöst, so löst sich auch nur der ursprünglich verklebte Teil der Befestigungseinrichtung 11. Der ursprünglich verklebte Teil kann eine Hälfte der Befestigungseinrichtung 11 sein. Der ursprünglich verklebte Teil kann einer der Schenkel 95a und 95b sein. Löst sich einer der Schenkel 95a und 95b aufgrund des Einsatzes des Licht- oder Wärmestrahlers 23, so kann sich die Befestigungseinrichtung 11 teilweise vom Rücken 13 lösen und auf diese Weise die Blutschlauchsatz-Komponente freigeben.

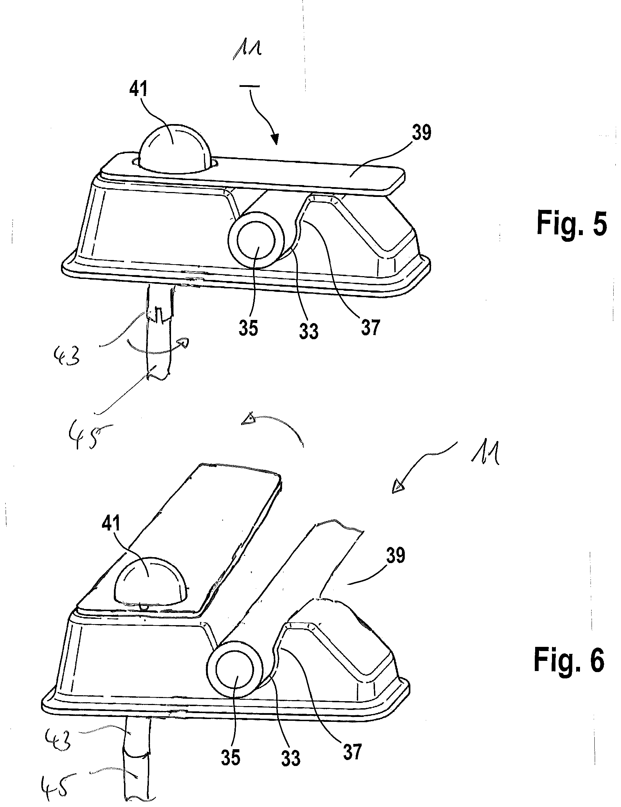

**Fig. 5** zeigt eine Befestigungseinrichtung 11 des erfindungsgemäßen Organizers 1 gemäß einer dritten Ausführungsform in perspektivischer Ansicht von vorne und mit einer Blutschlauchsatz-Komponente 35 als Beispiel einer Komponente im Sinne der vorliegenden Erfindung.

Die Befestigungseinrichtung 11 weist einen Aufnahmeabschnitt 33 zum lösbaren Aufnehmen eines Abschnitts der Blutschlauchsatz-Komponente 35, wobei der Aufnahmeabschnitt 33 optional geneigte Flächen 37 für eine einfachere Aufnahme der Blutschlauchsatz-Komponente 35 im Aufnahmeabschnitt 33 aufweist.

In Fig. 5 ist ferner eine Lasche 39 gezeigt, welche mittels einer Fixiereinrichtung 41 an der Befestigungseinrichtung 11 fixiert ist. Die Lasche 39 ist, wie im Vergleich mit Fig. 6 zu erkennen ist, mittels der Fixiereinrichtung 41 drehbar an der Befestigungseinrichtung 11 befestigt.

Die Lasche 39 verhindert aufgrund ihrer Anordnung über der BlutschlauchsatzKomponente 35 und deren Austrittsöffnung aus dem Kanal 97 ein ungewolltes Freigeben der Blutschlauchsatz-Komponente 35 aus dem Aufnahmeabschnitt 33. Sie lässt sich zur gewollten Entnahme der Blutschlauchsatz-Komponente 35 - wie in Fig. 6 dargestellt - um eine Achse der Fixiereinrichtung 41 drehen.

Die Fixiereinrichtung 41 ist rotationsfest sowohl mit der Lasche 39 als auch mit einem optionalen Verbinder 43 verbunden, welcher mit einer Vorrichtung 45 zum Freigeben von Komponenten verbindbar ist. Rotiert die Vorrichtung 45 zum Freigeben von Komponenten, veranlasst durch die Steuervorrichtung 350, den Verbinder 43, so dreht dieser die Lasche 39 zur Seite und gibt so die Blutschlauchsatz-Komponente 35 zur Entnahme frei. Dieser Zustand ist in Fig. 6 gezeigt.

**Fig. 6** zeigt die Befestigungseinrichtung 11 der Fig. 5 mit einer mittels der Vorrichtung zum Einwirken auf die Komponente geöffneten Lasche 39. Die BlutschlauchsatzKomponente 35 kann der Befestigungseinrichtung 11 - anders als in dem in Fig. 5 gezeigten geschlossenen Zustand der Befestigungseinrichtung 11 - manuell entnommen oder mittels eines in den Fig. 5 und 6 wiederum nicht gezeigten, optionalen Auswerfers 19 ausgeworfen werden.

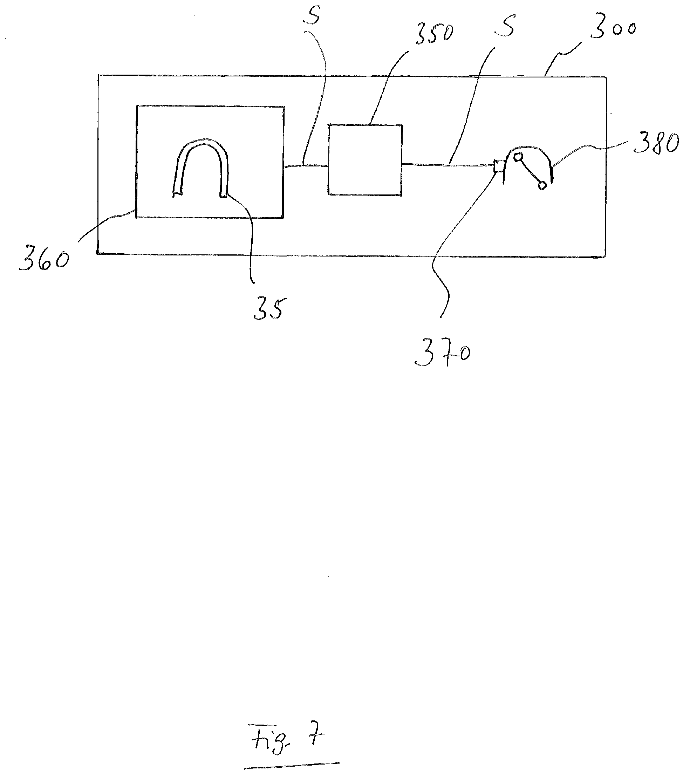

**Fig. 7** zeigt schematisch vereinfacht eine erfindungsgemäße Blutbehandlungsvorrichtung 300 in einer exemplarischen Ausführungsform.

Die Blutbehandlungsvorrichtung 300 weist die Steuervorrichtung 350 auf, welche in Signalverbindung S mit einem User Interface 360 und einer Detektionsvorrichtung 370 steht.

Das User Interface 360 zeigt dem Benutzer, etwa im Rahmen einer Anleitung zum Aufrüsten der Blutbehandlungsvorrichtung 300 mit Blutschlauchsatz-Komponenten eines Organizers 1, die Blutschlauchsatz-Komponente 35 als Komponente an, mit welcher die Blutbehandlungsvorrichtung 300 als nächstes aufzurüsten ist.

Die Detektionsvorrichtung 370 ist angeordnet und konfiguriert, um das erfolgte Einlegen der Blutschlauchsatz-Komponente 35 in die Blutpumpe 380 zu detektieren.

Erst wenn die Steuervorrichtung 350 mittels der Detektionsvorrichtung 370 feststellen kann, dass die Blutschlauchsatz-Komponente 35 auch tatsächlich in die Blutpumpe 380 eingelegt wurde, wird dem Benutzer mittels User Interface 360 die als nächstes zu verbindende Komponente (nicht gezeigt) dargestellt. Diese kann nach Detektion, dass die Blutschlauchsatz-Komponente 35 verbunden wurde, und/oder nachdem die nächste Komponente am User Interface 360 dargestellt wurde, freigegeben oder ausgeworfen werden.

### Bezugszeichenliste

- 1: Organizer
- 2: Rückgabeleitung, Komponente
- 3: Filtratleitung, Komponente
- 4: Dialysatleitung, Komponente
- 5: Substituatleitung; Komponente
- 6: Zugangsleitung, Komponente

- 2in: Filterkonnektor, Komponente
- 2out: Patientenkonnektor, Komponente
- 3in: Filterkonnektor, Komponente
- 3out: Konnektor, Komponente

- 4in: Konnektor, Komponente
- 4out: Filterkonnektor, Komponente
- 5in: Konnektor, Komponente
- 5out: Konnektor, Komponente
- 6in: Patientenkonnektor, Komponente
- 6out: Filterkonnektor, Komponente

- 11: Befestigungseinrichtungen
- 13: Rücken
- 15: Aufnahmeabschnitt
- 17: Öffnung
- 19: Auswerfer, Vorrichtung zum Auswerfen; Vorrichtung zum aktiven Trennen von Komponenten
- 20: Vorrichtung zum lösbaren Befestigen oder Fixieren des Organizers am Aufnahmeabschnitt
- 21: Klebeschicht
- 23: Licht- oder Wärmestrahler, Energiequelle oder -emitter zum Emittieren von Wellen, Licht und/oder Wärme
- 33: Aufnahmeabschnitt
- 35: Blutschlauchsatz-Komponente, Komponente, vorbestimmte erste Komponente, dargestellte oder bezeichnete Komponente
- 37: Flächen
- 39: Lasche
- 41: Fixiereinrichtung
- 43: Verbinder
- 45: Vorrichtung zum Freigeben von Komponenten
- 95a: Schenkel
- 95b: Schenkel
- 97: Kanal
- 99a: Vorsprung
- 99b: Vorsprung
- 101: Detektionsvorrichtung

- 300: Blutbehandlungsvorrichtung
- 350: Steuer- oder Regelvorrichtung
- 360: User Interface
- 370: Detektionsvorrichtung
- 380: Blutpumpe

- K: Weite des Kanals 97
- Q: Weite der Öffnung des Kanals 97
- S: Signalverbindung

## Patentansprüche

1. Blutbehandlungsvorrichtung (300), mit wenigstens je:
- einer Steuervorrichtung (350);
- einem User Inferface (360);
- einem Aufnahmeabschnitt (15) zum lösbaren Aufnehmen eines Organizers (1), wobei der Organizer (1) lösbar mit ihm verbundene Komponenten (2, 3, 4, 6, 35) für mittels der Blutbehandlungsvorrichtung (300) durchführbare, extrakorporale Blutbehandlungsoptionen, insbesondere Dialyseverfahren, trägt,
wobei die Blutbehandlungsvorrichtung (300), insbesondere im Bereich ihres Aufnahmeabschnitts (15) oder diesem zugeordnet, wenigstens eine Vorrichtung (20) zum lösbaren Befestigen oder Fixieren des Organizers (1) an der Blutbehandlungsvorrichtung (300) und wenigstens eine Vorrichtung (19, 23, 45) zum Einwirken auf Komponenten eines im oder am Aufnahmeabschnitt (15) lösbar aufgenommenen Organizers (1) aufweist,
**dadurch gekennzeichnet, dass**
die Vorrichtung zum Einwirken auf Komponenten (2, 3, 4, 6, 35) des Organizers (1) eine Vorrichtung (45) zum Freigeben von Komponenten des Organizers (1) zu deren manuellen Entnahme vom Organizer (1), eine Vorrichtung (19) zum aktiven Trennen von Komponenten vom Organizer (1) und/oder eine Vorrichtung zum Halten von Komponenten am Organizer (1) ist oder aufweist.

2. Blutbehandlungsvorrichtung (300) nach Anspruch 1, wobei die Vorrichtung (19, 23, 45) zum Einwirken auf Komponenten des Organizers (1) als wenigstens ein mechanisch wirkendes Bauteil ausgestaltet ist oder ein solches aufweist, wobei das mechanisch wirkende Bauteil insbesondere als eine Vorrichtung (19) zum Auswerfen, eine Vorrichtung zum Öffnen eines Riegels des Organizers (1), eine Drehvorrichtung zum Entriegeln eines Bajonettverschlusses oder einer Lasche (39) des Organizers (1), eine Vorrichtung zum Eröffnen eines Hinterschnitts des Organizers (1) und/oder eine Vorrichtung zum Spreizen oder Verklemmen zweier Abschnitte des Organizers (1) ausgestaltet ist oder eine solche aufweist.

3. Blutbehandlungsvorrichtung (300) nach einem der vorangegangenen Ansprüche, wobei die Vorrichtung (19, 23, 45) zum Einwirken auf Komponenten des Organizers (1) als eine Energiequelle (23) oder -emitter zum Emittieren von Wellen, Licht und/oder Wärme, und/oder als eine Magnetfeldquelle ausgestaltet ist oder eine solche aufweist.

4. Blutbehandlungsvorrichtung (300) nach einem der vorangegangenen Ansprüche, wobei die Steuervorrichtung (350) konfiguriert und/oder programmiert ist, die Vorrichtung (19, 23, 45) zum Einwirken auf Komponenten eines im Aufnahmeabschnitt (15) lösbar aufgenommenen Organizers (1) zu einem Einwirken auf Komponenten (2, 3, 4, 6, 35) zu aktivieren.

5. Blutbehandlungsvorrichtung (300) nach einem der vorangegangenen Ansprüche, wobei die Steuervorrichtung (350) konfiguriert und/oder programmiert ist, um auf Komponenten (2, 3, 4, 6, 35) des Organizers (1) in Abhängigkeit einer mittels User Interface (360) dargestellten oder bezeichneten Komponente (35) mittels der Vorrichtung (19, 23, 45) zum Einwirken auf Komponenten einzuwirken.

6. Blutbehandlungsvorrichtung (300) nach einem der vorangegangenen Ansprüche, wobei die Blutbehandlungsvorrichtung (300) wenigstens eine Detektionsvorrichtung (370) aufweist, welche mit der Steuervorrichtung (350) in Signalverbindung (S) steht und konfiguriert ist, eine vorliegende Verbindung einer vorbestimmten ersten Komponente (35) mit der Blutbehandlungsvorrichtung (300) zu detektieren;
wobei die Steuervorrichtung (350) konfiguriert und/oder programmiert ist, bei, oder bis zur, Detektion der erfolgten Verbindung der ersten vorbestimmten Komponente (35) auf eine vorbestimmte zweite Komponente mittels der Vorrichtung (19, 23, 45) zum Einwirken auf Komponenten vom Organizer (1) einzuwirken.

7. Blutbehandlungsvorrichtung (300) nach einem der vorangegangenen Ansprüche,
wobei die Blutbehandlungsvorrichtung (300) aufweist:
- wenigstens eine Detektionsvorrichtung (370), welche mit der Steuervorrichtung (350) in Signalverbindung (S) steht und konfiguriert ist, eine vorliegende Verbindung einer vorbestimmten ersten Komponente (35) mit der Blutbehandlungsvorrichtung (300) zu detektieren;
wobei die Steuervorrichtung (350) konfiguriert und/oder programmiert ist, um:
- den Benutzer mittels User Interface (360) zum Verbinden der Blutbehandlungsvorrichtung (300) mit einer vorbestimmten Komponente (2, 3, 4, 6, 35) des Organizers (1) aufzufordern;
- Signale der Detektionsvorrichtung hinsichtlich Detektion der erfolgten Verbindung der ersten vorbestimmten Komponente (35) auszuwerten; und
- bei, oder bis zur, Detektion der erfolgten Verbindung der ersten vorbestimmten Komponente (35) den Benutzer mittels User Interface (360) zum Verbinden der Blutbehandlungsvorrichtung (300) mit einer vorbestimmten weiteren Komponente des Organizers (1) aufzufordern und auf diese mittels Vorrichtung (19, 23, 45) zum Einwirken auf Komponenten des Organizers (1) einzuwirken.

8. Blutbehandlungsvorrichtung (300) nach einem der vorangegangenen Ansprüche, wobei die Blutbehandlungsvorrichtung (300) wenigstens eine Detektionsvorrichtung (370) aufweist, welche mit der Steuervorrichtung (350) in Signalverbindung steht und konfiguriert ist, eine vorliegende Verbindung einer zur Blutbehandlung erforderlichen Einrichtung mit der Blutbehandlungsvorrichtung (300) und/oder das erfolgte Ausführen einer zur Blutbehandlung erforderlichen, vorbestimmten Handlung/eines zur Blutbehandlung erforderlichen Vorgangs zu detektieren;
wobei die Steuervorrichtung (350) konfiguriert und/oder programmiert ist, um:
- Signale der Detektionsvorrichtung (370) hinsichtlich Detektion der erfolgten Verbindung der Einrichtung oder der erfolgten Handlung auszuwerten; und
- bei, oder bis zur, Detektion der erfolgten Verbindung der Einrichtung oder der erfolgten Handlung/des erfolgten Vorgangs, mittels der Vorrichtung (19, 23, 45) zum Einwirken auf Komponenten des Organizers (1), auf eine vorbestimmte Komponente (2, 3, 4, 6, 35) des Organizers (1) einzuwirken.

9. Blutbehandlungsvorrichtung (300) nach einem der vorangegangenen Ansprüche, lösbar mit einem Organizer (1) verbunden, welcher seinerseits lösbar mit ihm verbundene Komponenten (2, 3, 4, 6, 35) trägt.

10. Blutbehandlungsvorrichtung (300) nach einem der vorangegangenen Ansprüche, ausgestaltet als Hämodialysevorrichtung, Hämodiafiltrationsvorrichtung, Hämofiltrationsvorrichtung und/oder als Apharesevorrichtung.

11. Organizer (1) mit wenigstens einem, vorzugsweise steifen, Rücken (13) und wenigstens einer hieran angeordneten Befestigungseinrichtung (11), mittels welcher wenigstens eine als Blutschlauchsatzkomponente ausgestaltete Komponente (2, 3, 4, 6, 35) für die extrakorporale Blutbehandlung lösbar am Organizer (1) gehalten wird, wobei
- der Organizer, etwa der Rücken (13), und/oder die Befestigungseinrichtung (11) eine Öffnung (17) aufweist, durch welche eine Vorrichtung (19, 23, 45) zum Einwirken auf Komponenten, welche eine Vorrichtung der Blutbehandlungsvorrichtung (300) zum Freigeben von Komponenten des Organizers (1) zu deren manuellen Entnahme vom Organizer (1), eine Vorrichtung (19) zum aktiven Trennen von Komponenten vom Organizer (1) und/oder eine Vorrichtung zum Halten von Komponenten am Organizer (1) ist, mit der Komponente (2, 3, 4, 6, 35) und/oder der Befestigungseinrichtung (11) in Kontakt gebracht werden kann; und
- die Befestigungseinrichtung (11) oder eine der Komponenten (2, 3, 4, 6, 35) ein magnetisches Element aufweist.

## Claims

1. A blood treatment apparatus (300), having on each at least:
- one control device (350);
- one user interface (360);
- one receiving section (15) for releasably receiving an organizer (1), the organizer (1) being releasably connected to components (2, 3, 4, 6, 35) for extracorporeal blood treatment options, in particular dialysis methods, executable by the blood treatment apparatus (300),
wherein the blood treatment apparatus (300), in particular in the area of its receiving section (15), or in the area assigned to it, comprises at least one device (20) for detachably fastening or fixing the organizer (1) at the blood treatment apparatus (300) and at least one device (19, 23, 45) for acting on components of an organizer (1) releasably received in or at the receiving section (15),
**characterized in that**
the device for acting on components (2, 3, 4, 6, 35) of the organizer (1) is, or comprises, a device (45) for releasing components of the organizer (1) for their manual removal from the organizer (1), a device (19) for actively separating components from the organizer (1) and/or a device for holding components on the organizer (1).

2. The blood treatment apparatus (300) according to claim 1, wherein the device (19, 23, 45) for acting on components of the organizer (1) is at least designed as, or comprises, a mechanically operating element, the mechanically operating element being designed as, or comprising in particular,
a device (19) for ejecting,
a device for opening a lock of the organizer (1),
a rotating device for unlocking a bayonet lock or a lug (39) of the organizer (1),
a device for opening a back cut of the organizer (1) and/or a device for spreading apart or clamping two sections of the organizer (1).

3. The blood treatment apparatus (300) according to anyone of the preceding claims, wherein the device (19, 23, 45) for acting on components of the organizer (1) is designed as, or comprises, an energy source (23) or energy emitter for emitting waves, light and/or heat, and/or a magnetic field source.

4. The blood treatment apparatus (300) according to anyone of the preceding claims, wherein the control device (350) is configured and/or programmed to activate the device (19, 23, 45) for acting on components of an organizer (1), releasably received in the receiving section (15), in order to act on components (2, 3, 4, 6, 35).

5. The blood treatment apparatus (300) according to anyone of the preceding claims, wherein the control device (350) is configured and/or programmed to act on components (2, 3, 4, 6, 35) of the organizer (1) depending on a component (35) represented or designated by a user interface (360) by means of the device (19, 23, 45) for acting on components.

6. The blood treatment apparatus (300) according to anyone of the preceding claims, wherein the blood treatment apparatus (300) comprises at least a detection device (370) being in signal connection (S) with the control device (350) and being configured to detect an existing connection of a predetermined first component (35) with the blood treatment apparatus (300);
wherein the control device (350) is configured and/or programmed to act on a predetermined second component by means of the device (19, 23, 45) for acting on components of the organizer (1), during, or until detection of the made connection of the first predetermined component (35).

7. The blood treatment apparatus (300) according to anyone of the preceding claims, wherein the blood treatment apparatus (300) comprises:
- at least a detection device (370) being in signal connection (S) with the control device (350) and being configured to detect an existing connection of a predetermined first component (35) with the blood treatment apparatus (300);
wherein the control device (350) is configured and/or programmed to:
- request the user, by means of the user interface (360), to connect the blood treatment apparatus (300) with a predetermined component (2, 3, 4, 6, 35) of the organizer (1);
- evaluate signals of the detection device with regard to the detection of the made connection of the first predetermined component (35); and
- request the user, by means of the user interface (360), to connect the blood treatment apparatus (300) with a predetermined further component of the organizer (1) during, or until detection of the made connection of the first predetermined component (35), and act on the same by means of the device (19, 23, 45) for acting on components of the organizer (1).

8. The blood treatment apparatus (300) according to anyone of the preceding claims, wherein the blood treatment apparatus (300) comprises at least a detection device (370) in signal connection with the control device (350) and configured to detect an existing connection of a device required for blood treatment with the blood treatment apparatus (300) and/or to detect the completion of a predetermined action process required for blood treatment;
wherein the control device (350) is configured and/or programmed to:
- evaluate signals of the detection device (370) with regard to the detection of the made connection of the device or of the completed action; and
- act on a predetermined component (2, 3, 4, 6, 35) of the organizer (1) by means of the device (19, 23, 45) for acting on components of the organizer (1) during, or until detection of the completed connection of the device/the completed action of the completed process.

9. The blood treatment apparatus (300) according to anyone of the preceding claims, releasably connected to an organizer (1) which itself holds components (2, 3, 4, 6, 35) releasably connected to it.

10. The blood treatment apparatus (300) according to anyone of the preceding claims, designed as a hemodialysis apparatus, hemodiafiltration apparatus, hemofiltration apparatus and/or apheresis apparatus.

11. An organizer (1) comprising at least a, preferably rigid, rear (13) and at least a fastening device (11) arranged thereto, by means of which at least one component designed as a blood tubing set component (2, 3, 4, 6, 35) for extracorporeal blood treatment is releasably held to the organizer (1), wherein
- the organizer, such as the rear (13), and/or the fastening device (11) comprise/s an opening (17) through which a device (19, 23, 45) for acting on components, which is a device of the blood treatment apparatus (300) for releasing components of the organizer (1) for their manual removal from the organizer (1), a device (19) for actively separating components from the organizer (1) and/or a device for holding components on the organizer (1), can be brought into contact with the component (2, 3, 4, 6, 35) and/or the fastening device (11); and
- the fastening device (11), or one of the components (2, 3, 4, 6, 35), comprises a magnetic element.

## Revendications

1. Un appareil de traitement du sang (300), comprenant au moins:
- un dispositif de commande (350);
- une interface utilisateur (360);
- une section de réception (15) pour recevoir de manière amovible un organisateur (1), l'organisateur (1) portant des composants (2, 3, 4, 6, 35) lui étant reliés de manière amovible, pour des options de traitement du sang extracorporel, notamment des procédés de dialyse, pouvant être réalisés au moyen de l'appareil de traitement du sang (300),
où l'appareil de traitement du sang (300), notamment dans une zone de sa section de réception (15) ou lui étant affectée, comprend au moins un dispositif (20) destiné à attacher ou fixer de manière amovible l'organisateur (1) à l'appareil de traitement du sang (300) et au moins un dispositif (19, 23, 45) destiné à agir sur les composants d'un organisateur (1) reçu de manière amovible dans ou sur la section de réception (15),
**caractérisé en ce que**
le dispositif destiné à agir sur les composants (2, 3, 4, 6, 35) de l'organisateur (1) est, ou comprend, un dispositif (45) destiné à libérer les composants de l'organisateur (1) en vue de leur retrait manuel de l'organisateur (1), un dispositif (19) destiné à séparer activement les composants de l'organisateur (1) et/ou un dispositif destiné à maintenir les composants sur l'organisateur (1).

2. L'appareil de traitement du sang (300) selon la première revendication, où le dispositif (19, 23, 45) destiné à agir sur les composants de l'organisateur (1) est conçu comme, ou comprend, au moins un composant à action mécanique, le composant à action mécanique étant notamment conçu comme, ou comprenant,
un dispositif (19) d'éjection,
un dispositif d'ouverture d'un verrou de l'organisateur (1), un dispositif rotatif de déverrouillage d'une fermeture à baïonnette ou d'un loquet (39) de l'organisateur (1),
un dispositif d'ouverture d'une contre-dépouille de l'organisateur (1) et/ou
un dispositif d'écartement ou de serrage de deux sections de l'organisateur (1).

3. L'appareil de traitement du sang (300) selon l'une quelconque des revendications précédentes, où le dispositif (19, 23, 45) destiné à agir sur les composants de l'organisateur (1) est conçu comme, ou comprend, une source d'énergie (23) ou un émetteur d'énergie destiné à émettre des ondes, de la lumière et/ou de la chaleur, et/ou une source de champ magnétique.

4. L'appareil de traitement du sang (300) selon l'une quelconque des revendications précédentes, où le dispositif de commande (350) est configuré et/ou programmé pour activer le dispositif (19, 23, 45) destiné à agir sur les composants d'un organisateur (1) reçu de manière amovible dans la section de réception (15) afin d'agir sur des composants (2, 3, 4, 6, 35) .

5. L'appareil de traitement du sang (300) selon l'une quelconque des revendications précédentes, où le dispositif de commande (350) est configuré et/ou programmé pour agir sur des composants (2, 3, 4, 6, 35) de l'organisateur (1) en fonction d'un composant (35) représenté ou désigné par l'interface utilisateur (360) au moyen du dispositif (19, 23, 45) destiné à agir sur des composants.

6. L'appareil de traitement du sang (300) selon l'une quelconque des revendications précédentes, où l'appareil de traitement du sang (300) comprend au moins un dispositif de détection (370) étant en liaison de signal (S) avec le dispositif de commande (350) et étant configuré pour détecter l'existence d'une liaison entre un premier composant prédéterminé (35) et l'appareil de traitement du sang (300);
où le dispositif de commande (350) est configuré et/ou programmé pour agir sur un second composant prédéterminé au moyen du dispositif (19, 23, 45) destiné à agir sur des composants de l'organisateur (1), pendant, ou jusqu'à ce que l'établissement de la liaison du premier composant prédéterminé (35) soit détecté.

7. L'appareil de traitement du sang (300) selon l'une quelconque des revendications précédentes, où l'appareil de traitement du sang (300) comprend:
- au moins un dispositif de détection (370) étant en liaison de signal (S) avec le dispositif de commande (350) et étant configuré pour détecter l'existence d'une liaison entre un premier composant prédéterminé (35) et l'appareil de traitement du sang (300);
où le dispositif de commande (350) est configuré et/ou programmé pour:
- inviter l'utilisateur, au moyen de l'interface utilisateur (360), à relier l'appareil de traitement du sang (300) à un composant prédéterminé (2, 3, 4, 6, 35) de l'organisateur (1);
- évaluer des signaux du dispositif de détection relatifs à la détection de l'établissement de la liaison du premier composant prédéterminé (35); et
- inviter l'utilisateur, au moyen de l' interface utilisateur (360), à relier l'appareil de traitement du sang (300) à un autre composant prédéterminé de l'organisateur (1) pendant, ou jusqu'à ce que l'établissement de la liaison du premier composant prédéterminé (35) soit détecté, et agir sur celui-ci au moyen du dispositif (19, 23, 45) permettant d'agir sur les composants de l'organisateur (1).

8. L'appareil de traitement du sang (300) selon l'une quelconque des revendications précédentes, où l'appareil de traitement du sang (300) comprend au moins un dispositif de détection (370) en liaison de signal avec le dispositif de commande (350) et configuré pour détecter l'existence d'une liaison entre un équipement requis pour le traitement du sang et l'appareil de traitement du sang (300) et/ou l'exécution d'une action/d'un processus prédéterminé(e) requis(e) pour le traitement du sang;
où le dispositif de commande (350) est configuré et/ou programmé pour:
- évaluer des signaux du dispositif de détection (370) relatifs à la détection de l'établissement de la liaison de l'équipement ou de l'action effectuée; et
- agir sur un composant prédéterminé (2, 3, 4, 6, 35) de l'organisateur (1) au moyen du dispositif (19, 23, 45) destiné à agir sur les composants de l'organisateur (1) pendant, ou jusqu'à ce que l'établissement de la liaison de l'équipement ou l'exécution de l'action/du processus soit détecté(e).

9. L'appareil de traitement du sang (300) selon l'une quelconque des revendications précédentes, relié de manière amovible avec un organisateur (1) portant lui-même des composants (2, 3, 4, 6, 35) reliés de manière amovible à celui-ci.

10. L'appareil de traitement du sang (300) selon l'une quelconque des revendications précédentes, conçu sous la forme d'un appareil d'hémodialyse, d'un appareil d'hémodiafiltration, d'un appareil d'hémofiltration et/ou d'un appareil d'aphérèse.

11. Un organisateur (1) muni d'au moins un dossier (13), de préférence rigide, et d'au moins un équipement de fixation (11) agencé sur celui-ci, au moyen duquel au moins un composant conçu comme un composant (2, 3, 4, 6, 35) de set de tuyaux sanguins pour le traitement extracorporel du sang est maintenu de manière amovible sur l'organisateur (1), où
- l'organisateur, tel que le dossier (13), et/ou l'équipement de fixation (11) comprend/comprennent une ouverture (17) au travers de laquelle un dispositif (19, 23, 45) destiné à agir sur les composants, qui est un dispositif de l'appareil de traitement du sang (300) destiné à libérer les composants de l'organisateur (1) pour leur retrait manuel de l'organisateur (1), un dispositif (19) destiné à séparer activement des composants de l'organisateur (1) et/ou un dispositif destiné à maintenir des composants sur l'organisateur (1), peut être mis en contact avec le composant (2, 3, 4, 6, 35) et/ou avec l'équipement de fixation (11); et où
- le dispositif de fixation (11), ou un des composants (2, 3, 4, 6, 35), comprend un élément magnétique.
